# EUROPEAN PATENT APPLICATION

(11) **EP 1 930 327 A1**
(43) Date of publication of application: **11.06.2008**
(21) Application number: 06797201.8
(22) Date of filing: 31.08.2006
(51) Int. Cl.: C07D 303/16, C07D 301/30, C07D 303/24, C07D 305/06

(54) **ADAMANTANE DERIVATIVE, RESIN COMPOSITION CONTAINING SAME, AND OPTOELECTRONIC COMPONENT USING SAME**

(30) Priority: 01.09.2005 JP 2005253833
(71) Applicant: IDEMITSU KOSAN CO., LTD., Tokyo 100-8321 (JP)
(72) Inventor: ITO, Hajime, Ichihara-shi, Chiba 299-0193 (JP)
(74) Representative: Vossius & Partner
(86) International application number: PCT/JP2006/317241
(87) International publication number: WO 2007/026828

(57) **Abstract**

Disclosed is an adamantane derivative which enables to obtain a cured product which is excellent in optical characteristics such as transparency and light resistance, long-term heat resistance, and electrical characteristics such as dielectric constant. Also disclosed are a resin composition containing such an adamantane derivative, and a sealing agent for electronic circuits and an optoelectronic member respectively using those. Specifically disclosed are an adamantane derivative represented by the general formula (I) below, a resin composition containing such an adamantane derivative, a sealing agent for electronic circuits and an optoelectronic member respectively using such a resin composition, and an adhesive of those. (I) In the general formula (I), Z represents a group selected from the groups represented by the general formula (II) or (III) below (wherein R₁ represents a hydrogen atom or an alkyl group having 1-4 carbon atoms); X represents a linking group which may contain an oxygen atom and a sulfur atom and is composed of an (m + 1)-valent hydrocarbon, and a part or all of the hydrogen atoms in a hydrocarbon as the linking group may be replaced with fluorine atoms; Y represents a group selected from -CO₂- and -O-; W represents a group selected from a hydrogen atom, a halogen atom, a hydrocarbon group, a halogen-substituted hydrocarbon group, a cyclic hydrocarbon group, a halogen-substituted cyclic hydrocarbon group, a hydroxyl group, a carboxyl group and =O formed from two Ws combined together; m represents an integer of 2-4; n represents an integer of 1-4; and k represents an integer from 0 to 16 - n.

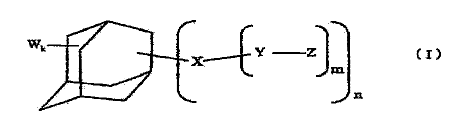

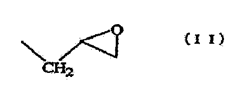

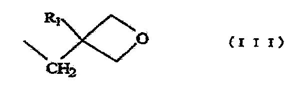

## Description

### Technical Field

The present invention relates to a novel adamantane derivative, a resin composition containing the derivative, and a sealant for an electronic circuit and an optoelectronic component respectively using the composition.

### Background Art

An adamantane contains 4 cyclohexane rings condensed to form a cage skeleton, and is highly symmetric and stable. Its derivatives show specific performances, thus are known to be useful as raw materials for a pharmaceutical raw material, a high performance industrial material and the like. An adamantane has, for example, optical characteristics, heat resistance and the like, and therefore attempts have been made to use it for an optical disk substrate, an optical fiber, a lens and the like (for example, refer to Patent Documents 1 and 2). Furthermore, there have been attempts to use an adamantane ester as a raw resin material for a photoresist by utilizing its acid-sensitive property, dry etching resistance, UV light transparency and the like (for example, refer to Patent Document 3).
In recent years, in order to achieve higher performance or improvement of an optical/electronic component, studies are progressing for higher precision, wider viewing angle, and enhanced image quality of a flat panel display using a liquid crystal, an organic electroluminescence (EL) device and the like, for higher intensity/shorter wavelength and whitening of a light source using such optical semiconductors as a light emitting diode (LED) and the like, and further for higher frequency of an electronic circuit and for an optical circuit/communication, and others.
As a method for the improvement, a basic material of a light emitting material and the like used for a liquid crystal material and an organic EL device has been investigated and developed. The investigation has also been done for higher performance of a resin used along with those materials as a coating material, a sealant and the like. As a resin for a coating material of an optical/electronic component and for a sealant, many kinds of thermosetting resin, light-curable resin, or thermoplastic resin have been applied. They have been applied in accordance with their respective characteristics in heat resistance, transparency, solubility, adhesiveness, and others of each resin.
In the field of LED, which is advanced in terms of high performance, an illumination, a light and the like by using a white LED comprising light emitting devices of near ultraviolet and blue lights have been proposed and developed for practical use. In addition, it is expected that they will be developed to be used for a home lighting and an automobile in the future. An LED device is sealed by a resin containing a fluorescent material in an inorganic semiconductor. For such applications, thermosetting resins such as conventional bisphenol A epoxy resin and the like have limitation in heat resistance and light resistance, thus a sealant fulfilling those required characteristics is desired (for instance, refer to Non-Patent Document 1). As its improved product, a hydrogenated alicyclic epoxy compound and the like having low absorption in a shorter wavelength range have been proposed, but their heat resistance is lowered on the contrary.

Furthermore, in the display field, an organic EL device of small size, high precision, and energy saving is used, and such type as top-emission is employed. Accordingly, a sealing resin itself is required, for the use in an organic EL device, to have further improved transparency, light resistance, heat-resistance, mechanical strength and others in addition to such functions as gas barrier, adhesion of conventional sealing boards such as stainless steel and the like to a glass substrate, and others (for instance, refer to Non-Patent Document 2).
Furthermore, in an electronic circuit integrated with a semiconductor and the like, as a computerized society progresses, the increase in volume of information and communication speed and the miniaturization of a device have been achieved, thus further miniaturization, integration, and an increase in frequency are necessary. Furthermore, an optical circuit using an optical waveguide and the like that enable further high speed processing has also been investigated. Such conventional resins as a bisphenol A epoxy resin and the like, which have been used as a sealing resin, a film for these uses, or a resin for a lens, have problems such as high dielectric constant in an electronic circuit, a decrease in transparency because of light absorption by an aromatic ring in an optical waveguide and an LED sealant, yellowing of a resin due to decaying, and the like.
For these reasons, there has been used an alicyclic epoxy resin having no aromatic rings which is prepared by such methods as epoxidizing a carbon-carbon double bond, reacting a polyvalent alicyclic alcohol with epichlorohydrin, hydrogenating an aromatic ring of an aromatic epoxy resin and the like. However, a cured cyclic aliphatic epoxy resin (such as 3,4-epoxycyclohexylmethyl-3',4'-epoxycyclohexanecarboxylate and the like) that is prepared by epoxidizing a carbon-carbon double bond is brittle and insufficient in impact strength and in adhesion strength to a metal and the like. Also, as an alicyclic epoxy resin that is prepared by reacting a polyvalent alicyclic alcohol with epichlorohydrin or by hydrogenating an aromatic ring of an aromatic epoxy resin, a hydrogenated bisphenol A epoxy resin is known, but it is inferior to a bisphenol epoxy resin in heat resistance. Also, in an epoxy resin that is prepared by reacting a hydrogenated bisphenol A with epichlorohydrin there are problems of deterioration of electric characteristics such as an increase in dielectric constant and the like since a chlorine component tends to remain in the product during its production.
Furthermore, an epoxy resin using dicyclopentadiene dimethanol as an alicyclic alcohol is proposed (for instance, refer to Patent Documents 4 and 5), but there is no description of such information as heat resistance, and transparency, long-term reliability/durability and the like that are required for optical and electronic materials, though it has effects of decrease in water absorption and of lowered stress.
A polymer compound having an adamantane skeleton is excellent in heat resistance and, for instance, a polyester, a polycarbonate and the like, all using an adamantane diol, are known as such polymers. Further, compositions containing 1,3-bis(glycidyloxyphenyl)adamantane and 2,2-bis(glycidyloxyphenyl)adamantane are proposed (for instance, refer to Patent Documents 6 and 7). However, although lowering of dielectric constant and improvement of transparency are observed as compared with a bisphenol A epoxy resin, it may not be said that these effects are sufficient since they contain an aromatic ring. Further, compositions containing glycidyl ethers of an adamantane diol and of adamantane dimethanol are proposed (for instance, refer to Patent Document 8). In this Document, heat resistance and transparency are reported to be improved, but their specific values are not shown, nor is disclosed long-term reliability/durability at all. Also, a composition containing a glycidyl ester of an adamantane dicarboxylic acid is proposed, but there is no description of transparency, mechanical characteristics, long-term reliability/durability and the like, though there are data showing an increase in heat resistance (for instance, refer to Patent Document 6).

Patent Document 1: Japanese Patent Laid-Open Publication No. H6-305044
Patent Document 2: Japanese Patent Laid-Open Publication No. H9-302077
Patent Document 3: Japanese Patent Laid-Open Publication No. H4-39665
Non-Patent Document 1: Monthly "Material Stage" June 2003, pp.20-24, published by Technical Information Institute Co., Ltd.
Non-Patent Document 2: Monthly "Material Stage" March 2003, pp.52-64, published by Technical Information Institute Co., Ltd.
Patent Document 4: Japanese Patent Laid-Open Publication No. 2001-81286
Patent Document 5: Japanese Patent Laid-Open Publication No. 2004-83732
Patent Document 6: Japanese Patent Laid-Open Publication No. 2003-321530
Patent Document 7: Japanese Patent Laid-Open Publication No. H10-130371
Patent Document 8: International Laid-Open Publication No. WO2004/113313

### Disclosure of the Invention

### Problems to be Solved by the Invention

In view of the above-mentioned circumstances, an object of the present invention is to provide an adamantane derivative giving a cured product that is excellent in optical characteristics such as transparency and light resistance, long-term heat resistance, and electric characteristics such as dielectric constant and the like, useful as a sealant for electronic circuits such as an optical semiconductor, an organic EL device and the like, and as optoelectronic components such as an optical waveguide, a lens for optical communication, an optical film and the like. Also, an object of the present invention is to provide a resin composition containing the derivative, and an optoelectronic component respectively using the composition and a sealant for an electronic circuit. Means for Solving the Problems

The present inventors investigated extensively to achieve the above objectives, and as a result, have found that a resin composition giving a cured product suitable as a sealant for an electronic circuit and an optoelectronic component is obtained by using a specific adamantane derivative. The present invention was accomplished based on these findings.
Namely, the present invention provides a following adamantane derivative, a resin composition containing the derivative, and an optoelectronic component using the composition.
1. An adamantane derivative represented by the following general formula (I).

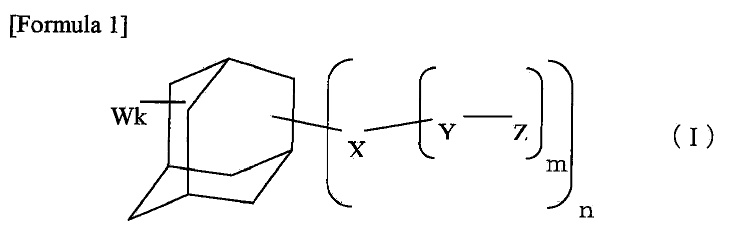

[In the formula, Z represents a group selected from the groups represented by the following general formula (II),

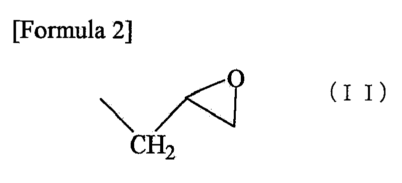

or the following general formula (III)

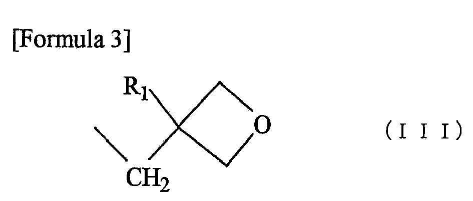

(wherein R₁ represents a hydrogen atom or an alkyl group having 1 to 4 carbon atoms). X represents a linking group that may contain an oxygen atom and / or a sulfur atom and is composed of an (m + 1)-valent hydrocarbon. And a part or all of the hydrogen atoms in the hydrocarbon as the linking group may be replaced with fluorine atoms. Y represents a group selected from -CO₂- and -O-. W represents a group selected from a hydrogen atom, a halogen atom, a hydrocarbon group, a hydroxyl group, a carboxyl group, and =O formed from two Ws combined together; m represents an integer of 2 to 4; n represents an integer of 1 to 4; and k represents an integer of 0 to 16-n].
2. The adamantane derivative according to the above 1, wherein n is 1.
3. The adamantane derivative according to the above 1 or 2, wherein the linkage between an adamantane skeleton and X is made by an ether bond.
4. A method for producing the adamantane derivatives according to the above 1, wherein non-aromatic adamantanes and an alkyl-containing cyclic ether compound are reacted in the presence of a base catalyst.
5. A method for producing the adamantane derivatives according to the above 1, wherein non-aromatic adamantanes and an epihalohydrin compound are reacted by an addition reaction under an acidic condition, followed by a reaction in the presence of a base catalyst.
6. A method for producing the adamantane derivative according to the above 1, wherein a corresponding aromatic adamantane derivative is ring-hydrogenated in the presence of a rhodium catalyst or a ruthenium catalyst.
7. An oligomer of an adamantane derivative represented by the following general formula (IV),

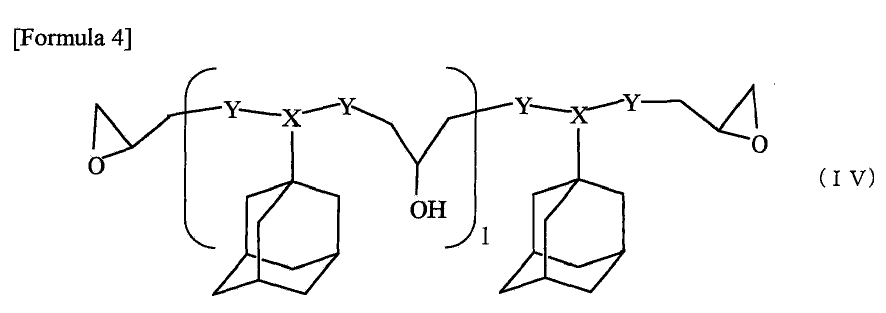

[In the formula, X and Y represent the same as above; I represents an integer of 1 to 4]

or by the following general formula (V).

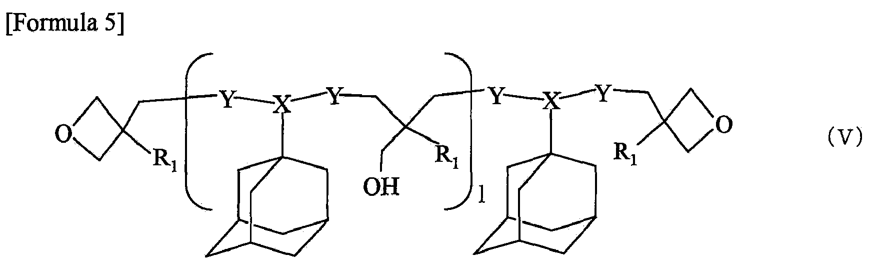

[In the formula, X, Y, and R₁ represent the same as before; 1 represents an integer of 1 to 4]

8. A resin composition obtained by curing the adamantane derivative according to any of the above 1 to 3 and 7 by using a curing agent for an epoxy resin.
9. The resin composition according to the above 8, wherein the curing agent for an epoxy resin is an acid anhydride compound and/or a cationic polymerization initiator.
10. An optoelectronic component using the adamantane derivative according to any of the above 1 to 3 and 7.
11. The optoelectronic component using the resin composition according to the above 8 or 9.
12. A sealant for an electronic circuit using the adamantane derivative according to any of the above 1 to 3 and 7.
13. A sealant for an electronic circuit using the resin composition according to the above 8 or 9.

### Effects of the Invention

A resin composition containing an adamantane derivative of the present invention gives a cured product which is excellent in optical characteristics such as transparency and light resistance, long-term heat resistance, and electric characteristics such as dielectric constant, and is suitable as a sealant for electronic circuits such as an optical semiconductor, an organic EL device and the like, as optoelectronic components such as an optical waveguide, a lens for optical communication, an optical film and the like, and as an adhesive for them.

### Best Mode for Carrying Out the Invention

### [An adamantane derivative]

An adamantane derivative of the present invention is represented by the following general formula (I).

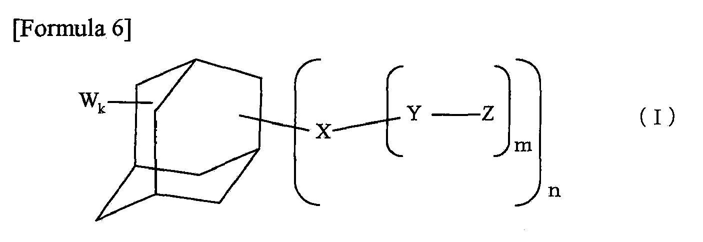

In the above general formula (I), X represents a linking group composed of an (m + 1)-valent, namely tri- to penta-valent hydrocarbon. A part or all of the hydrogen atoms in the hydrocarbon as the linking group may be replaced with fluorine atoms, and the linking group may contain an oxygen atom and/or a sulfur atom. And the valency of the linking group, namely the valency of a free atom may be present at any position of the hydrocarbon group, which may be linear or branched and may have a substituent group. When plural Xs are introduced, each X may be the same or different.
When X is a linking group composed of a tri-valent hydrocarbon, examples of X include such tri-valent alkanetriyl groups as a methylidine group, an ethylidine group, a propanylidine group, a butanylidine group, a I-ethanyl-2-ylidene group, a 1,2,3-propanetriyl group, a I-propanyl-3-ylidene group, a 1,2,4-butanetriyl group, a 1-butanyl-4-ylidene group, a 1,2,5-pentanetriyl group, a 1,3,5-pentanetriyl group, a I-pentanyl-5-ylidene group, a 1,2,6-hexanetriyl group, a 1,3,6-hexanetriyl group, a I-hexanyl-6-ylidene group, a 2-propanyl-1-ylidene group, a 2-methylene-1,3-propanediyl group, a 1-propanyl-3-ylidene group, a 3-butanyl-1-ylidene group, a 1,2,3-butanetriyl group, a 1-butanyl-2-ylidene group, a 2-methyl-1-propanyl-3-ylidene group, a 2-methyl-1,2,3-propanetriyl group, a 2-ethyl-1,2,3-propanetriyl group, a 2-butanyl-1-ylidene group, a 2-butanyl-3-ylidene group, a 2-butanyl-4-ylidene group, a 1,3,7-heptanetriyl group, a 1,4,8-octanetriyl group, a 1,5,9-nonanetriyl group, a 1,5,10-decanetriyl group, a 1,6,11-undecanetriyl group, a 1,6,12-dodecanetriyl group and the like; such cycloalkanetriyl groups as a 1,2,3-cyclopentanetriyl group, a 1,2,5-cyclopentanetriyl group, a 1-cyclopentyl-2-ylidene group, a 1-cyclopentyl-3-ylidene group, a 1,2,3-cyclohexanetriyl group, a 1,2,4-cyclohexanetriyl group, a 1,3,5-cyclohexanetriyl group, a 1-cyclohexyl-2-ylidene group, a 1-cyclohexyl-3-ylidene group, a 1-cyclohexyl-4-ylidene group, a cyclohexylmethylidine group, a 4-cyclohexylenemethylene group, a 1-cyclohexyl-2-ethanyl-1-ylidene group and the like; such arylenetriyl groups as a 1,3,5-benzenetriyl group, a benzylidine group, a 2-phenyl-1,2,3-propanetriyl group, a naphthalenetriyl group and the like; and the like.

When X is a linking group composed of a tetra-valent hydrocarbon, specific examples of X include, for example, a propane-1,3-diylidene group, a 1,3-propanediyl-2-ylidene group, a butane-1,4-diylidene group, a pentane-1,5-diylidene group, a hexane-1,6-diylidene group, a hepatane-1,7-diylidene group, an octane-1,8-diylidene group, a nonane-1,9-diylidene group, a decane-1,10-diylidene group, an undecane-1,11-diylidene group, a dodecane-1,12-diylidene group, a 1,2,3,4-cyclohexanetetrayl group, a cyclohexane-1,3-diylidene group, a cyclopentane-1,3-diylidene group, a 1,2,4,5-benzenetetrayl group, a naphthalenetetrayl group and the like.

When X is a linking group composed of a penta-valent hydrocarbon, specific examples of X include, for example, a I-ethylidine-2-ylidene group, a 2-propaneyl-1,3-diylidene group, a 2-butaneyl-1,4-diylidene group, a 2-pentaneyl-1,5-diylidene group, a 2-hexaneyl-1,6-diylidene group, a 2-heptaneyl-1,7-diylidene group, a 2-octaneyl-1,8-diylidene group, a 2-nonaneyl-1,9-diylidene group, a 2-decaneyl-1,10-diylidene group, a 2-undecaneyl-1,11-diylidene group, a 2-dodecaneyl-1,12-diylidene group, a 1,2,3,4,5-cyclohexanepentayl group, a 2-cyclohexaneyl-1,3-diylidene group, a 2-cyclopentaneyl-1,3-diylidene group, a 1,2,4,5,6-benzenepentayl group, a naphthalenepentayl group and the like.
Also, a linkage between an adamantane skeleton and a linking group X is preferably made by an ether bond. For example, when the hydrocarbon group having tri- to penta-valency described above is represented by R₂, the linking group X is represented by -O-R₂-, forming an ether bond between an adamantane skeleton and an oxygen atom. The linkage between an adamantane skeleton and a linking group X is preferably made by an ether bond in view of easy synthesis from relatively cheap raw materials.

Y represents either a -CO₂- group or a -O- group. When plural Ys are introduced, each Y may be the same or different.
W represents a group selected from a hydrogen atom, a halogen atom, a hydrocarbon group, a hydroxyl group, a carboxyl group, and =O formed from two Ws combined together. Specific examples of the hydrocarbon groups represented by W include an alkyl group, an alkoxy group, an aromatic hydrocarbon group, an aryloxy group, an aralkyl group, an alkenyl group, a cycloalkyl group, an alkylamino group, an arylamino group, an aralkylamino group and the like. These hydrocarbon groups may be linear, branched, or cyclic, and may be substituted. Specific examples include a methyl group, an ethyl group, a propyl group, a butyl group, a cyclohexyl group, a phenyl group, a methoxy group, an ethoxy group, a cyclopentyl group, a methylcyclopentyl group, a cyclohexyl group, a methylcyclohexyl group, an ethylcyclohexyl group and the like. A halogen-substituted hydrocarbon group is those in which one or more of hydrogen atom(s) of the above hydrocarbon is (are) replaced by (a) halogen atom(s), and examples of them include a trifluoromethyl group, a fluorocyclopentyl group, a fluorocyclohexyl group, a trifluoromethylcyclopentyl group, a trifluoromethylcyclohexyl group and the like. Examples of the halogen atoms are fluorine, chlorine, bromine, and iodine. When plural Ws are introduced, each W may be the same or different.
Here, m represents an integer of 2 to 4; n represents an integer of 1 to 4; k represents an integer of 0 to 16-n. From the viewpoint of synthesis by using a cheaper raw material, n is preferably 1.

In the above general formula (I), Z represents a group selected from the groups represented by the following general formula (II)

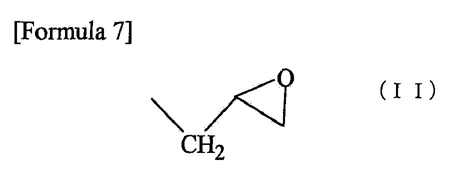

or the following general formula (III).

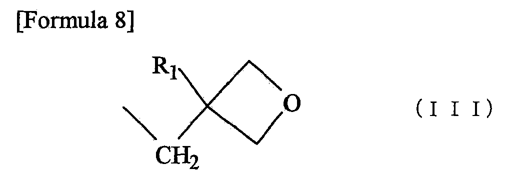

In the formula, R₁ represents a hydrogen atom or an alkyl group having 1 to 4 carbon atoms. The alkyl group may be linear, branched, or cyclic, and may be substituted. Specific examples include a methyl group, an ethyl group, a n-propyl group, an isopropyl group, a n-butyl group, a sec-butyl group, a tert-butyl group and the like. Further, when plural Zs are introduced, each Z may be the same or different.

Further, an adamantane derivative of the present invention includes an oligomer of an adamantane derivative represented by the following general formula (IV) or the general formula (V).

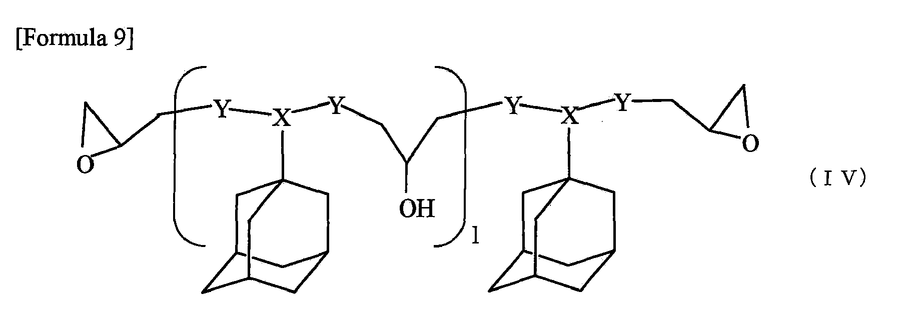

In the above general formula (IV), X and Y represent the same as mentioned above.
In the formula, I represents an integer of 1 to 4.

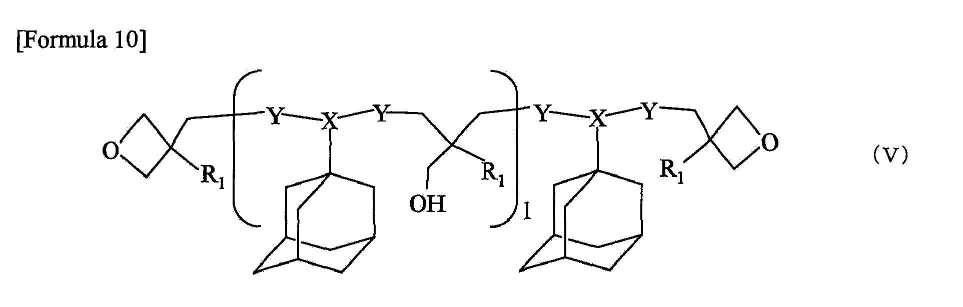

In the above general formula (V), X, Y, and R₁ represent the same as the above-mentioned. In the formula, 1 represents an integer of 1 to 4.

### [Synthesis method (1) of an adamantane derivative represented by the general formula (I)]

An adamantane derivative represented by the above general formula (I) may be synthesized by reacting a non-aromatic adamantane with an alkyl-containing cyclic ether compound in the presence of a base catalyst (hereinafter sometimes referred to as "Synthesis Method (1)" for short).
Examples of the non-aromatic adamantanes include 1-adamantylmalonic acid, 1-adamantylsuccinic acid, 1-adamantyloxymalonic acid, 1-admantyloxysuccinic acid, 2-(1-adamantyl)propane-1,3-diol, 2-(1-adamantyl)butane-1,4-diol, 2-(1-adamantyloxy)propane-1,3-diol, 3-(1-adamantyloxy)propane-1,2-diol, 2-(1-adamantyloxy)butane-1,4-diol, 1,3-bis(2,3-dihydroxy-1-propyoxy)adamantane, 2-(1-adamantyloxymethyl)-2-methylpropane-1,3-diol, 2-(1-adamantyloxymethyl)-2-ethylpropane-1,3-diol, 2-(1-adamantyloxymethyl)-2-hydroxymethylpropane-1,3-diol and the like.

Examples of the alkyl-containing cyclic ether compounds include those represented by the following general formula.

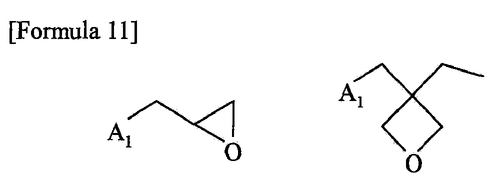

In the formula, A₁ represents a chlorine atom, a bromine atom, an iodine atom, a p-toluenesulfonic acid group, or a methylsulfonic acid group. Specific examples include epichlorohydrin, epibromohydrin, epiiodohydrin, 3-chloromethyl-3-methyloxetane, 3-methylsulfonate-3-methyloxetane and the like.

A reaction between a non-aromatic adamantane and an alkyl-containing cyclic ether compound as mentioned above is carried out usually at a temperature of about 0 to about 200°C, and preferably at 20 to 150°C. When the reaction temperature is within the above range, the reaction rate does not decrease and remains moderate, thus the reaction time is shortened and product coloring may be suppressed. Applied absolute pressure at the reaction is about 0.01 to about 10 MPa, and preferably normal pressure to 1 MPa. When the reaction pressure is within the above range, special equipment is not necessary as the safety is secured, thus it is useful from an industrial viewpoint. The reaction time is usually about 1 minute to about 24 hours, and preferably 1 to 10 hours. When the reaction time is within the above range, an amount of an unreacted raw material may be reduced, leading to increase in the reaction yield and to suppress coloring of a product and the formation of a by-product.
Further, an alkyl-containing cyclic ether compound is reacted with a non-aromatic adamantane derivative with the amount of the former being 1- to 20-fold equivalent, and preferably 2- to 10-fold equivalent, relative to an equivalent of carboxyl group (or a hydroxyl group) of the latter. When the amounts are within the above range, an amount of an unreacted raw material may be reduced, leading to increase in the reaction yield and to suppress slowing of the reaction rate and the formation of a by-product. Further, the reaction may be repeatedly carried out under the same conditions more than once until intended conversion is reached.

The above reaction is usually carried out in the presence of a base catalyst. Examples of the base catalysts include sodium amide, triethylamine, tributylamine, trioctylamine, pyridine, N,N-dimethylaniline, 1,5-diazabicyclo[4.3.0]nonene-5 (DBN), 1,8-diazabicyclo[5.4.0]undecene-7 (DBU), tetramethylammonium chloride, tetramethylammonium bromide, tetraethylammonium chloride, tetraethylammonium bromide, sodium hydroxide, potassium hydroxide, sodium hydride, sodium phosphate, potassium phosphate, sodium carbonate, potassium carbonate, silver oxide, sodium methoxide, potassium t-butoxide and the like.
The ratio of a base catalyst used relative to a hydroxyl group and a carboxyl group of a raw material monomer is about 1.0 to about 5.0 equivalents, and preferably 1.0 to 2.0 equivalents. When the ratio of a base catalyst used is within the above range, there is no residual alkyl-containing cyclic ether compound not subjected to a glycidyl formation reaction, thus the effect of catalyst used is sufficiently realized.

Further, in the above reaction, it is preferable to use a phase-transfer catalyst. As the phase-transfer catalyst, there is no particular restriction and any catalyst that is generally used may be used, and a quaternary ammonium salt, a crown ether, a phosphonium salt, an amine and the like may be cited as such examples. Specifically preferred catalysts, from the point of view of availability and easy handling, include quaternary ammonium salts such as tetramethylammonium chloride, tetraethylammonium bromide, benzyltrimethylammonium chloride, benzyltriethylammonium chloride, benzyltributylammonium chloride, tetrabutylammonium hydrogen sulfate and the like.

The reaction is carried out in the absence or presence of a solvent. As the solvent, a solvent having 0.5% by mass or more, and preferably 5% by mass or more of solubility of the above non-aromatic adamantane in it is used. The amount of the solvent to be used is such that the concentration of the above non-aromatic adamantane in it is 0.5% by mass or more, and preferably 5% by mass or more. Here, the above-mentioned adamantane may exist in the state of suspension but preferably in the state of solution. Specific examples of the solvents include hexane, heptane, toluene, dimethylformamide (DMF), N,N-dimethylacetamide (DMAc), dimethylsulfoxide (DMSO), ethyl acetate, diethyl ether, tetrahydrofuran and the like. These may be used singly or in a combination of two or more kinds.
A reaction product may be purified by distillation, crystallization, column-separation and the like, and the purification method may be selected depending on the nature of the products and the kind of impurities.

### [Synthesis method (2) for an adamantane derivative represented by the general formula (I)]

An adamantane derivative represented by the above general formula (I) may also be synthesized by reacting a non-aromatic adamantane with an epihalohydrin compound by addition reaction under an acidic condition, followed by further reaction in the presence of a base catalyst (hereinafter sometimes referred to as "Synthesis Method (2)" for short). As a non-aromatic adamantane derivative, the above-mentioned adamantanes may be cited.

As an epihalohydrin compound, the compounds represented by the following general formula may be cited.

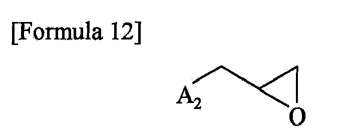

In the formula, A₂ represents a chlorine atom, a bromine atom, and an iodine atom. Specific examples include epichlorohydrin, epibromohydrin, epiiodohydrin and the like.

The addition reaction between a non-aromatic adamantane and an epihalohydrin as mentioned above is carried out usually at a temperature of about 0 to about 100°C, and preferably at 20 to 85°C. When the reaction temperature is within the above range, the reaction rate does not decrease and remains moderate, thus the reaction time is shortened and the formation of a by-product derived from a halogen-containing substance may be suppressed. Applied absolute pressure at the reaction is about 0.01 to about 10 MPa, and preferably normal pressure to 1 MPa. When the reaction pressure is within the above range, special equipment is not necessary as the safety is secured, thus it is useful from an industrial viewpoint. The reaction time is usually about 1 minute to about 24 hours, and preferably 30 minutes to 3 hours. When the reaction time is within the above range, amount of an unreacted raw material may be reduced, leading to increase in the reaction yield and manufacturing efficiency.
Further, an epihalohydrin is reacted with a non-aromatic adamantane derivative with the amount of the former being 0.9- to 1.5-fold equivalent, and preferably 1.0- to 1.2-fold equivalent, relative to an equivalent of carboxyl group (or a hydroxyl group) of the latter. When the amounts are within the above range, the reaction yield is increased and the formation of a chlorine-containing substance may be suppressed.

The above addition reaction is usually carried out in the presence of an acid catalyst. Generally used catalyst may be used, and specific examples include such mineral acids as sulfuric acid, hydrochloric acid, nitric acid, boron trifluoride, tin tetrachloride, hydrobromic acid, perchloric acid and the like, such sulfonic acids as p-toluenesulfonic acid, benzenesulfonic acid and the like, such carboxylic acids as oxalic acid, succinic acid, malonic acid, monochloroacetic acid, dichloroacetic acid and the like, and others.
The ratio of an acid catalyst used relative to a total of raw materials is about 0.1 to about 20% by mol, and preferably 0.5 to 10% by mol. Within the above range, shortening of the reaction time may be expected and the formation of a by-product derived from a halogen-containing substance may be suppressed.

The above addition reaction is carried out in the absence or presence of a solvent. As the solvent, a solvent having 0.5% by mass or more, and preferably 5% by mass or more of solubility of the above non-aromatic adamantane in it is used. The amount of the solvent to be used is such that the concentration of the above non-aromatic adamantane in it is 0.5% by mass or more, and preferably 5% by mass or more, wherein the adamantane may exist in the state of suspension but preferably in the state of solution. Specific examples of the solvents include hexane, heptane, toluene, dimethylformamide (DMF), N,N-dimethylacetamide (DMAc), dimethylsulfoxide (DMSO), ethyl acetate, diethyl ether, tetrahydrofuran and the like. These may be used singly or in a combination of two or more kinds.

After the above addition reaction, a ring-closure reaction is carried out usually in the presence of a base catalyst. Examples of the base catalysts include sodium hydroxide, potassium hydroxide, sodium hydride, sodium phosphate, potassium phosphate, sodium carbonate, potassium carbonate and the like.
The amount of a base catalyst used, other than the amount consumed for neutralization of an acid catalyst used in the above addition reaction, is 1 to 2 equivalents, and preferably 1 to 1.5 equivalents, relative to a hydroxyl group and a carboxyl group of the raw material. When the ratio of a catalyst used is within the above range, the hydration reaction to give glycidyl ether is suppressed, thus the ring-closure reaction proceeds sufficiently.

The above ring-closure reaction is carried out usually at a temperature of about 20 to about 100°C, and preferably at 30 to 80°C. When the reaction temperature is within the above range, the reaction rate does not decrease and remains moderate, thus the reaction time is shortened and coloring of a reaction product may be suppressed. Applied absolute pressure at the reaction is about 0.01 to about 10 MPa, and preferably normal pressure to 1 MPa. When the reaction pressure is within the above range, special equipment is not necessary as the safety is secured, thus it is useful from an industrial viewpoint. The reaction time is usually about 1 minute to about 24 hours, and preferably 30 minutes to 10 hours. When the reaction time is within the above range, an amount of an unreacted raw material may be reduced, leading to increase in the reaction yield and manufacturing efficiency.
The reaction is carried out in the absence or presence of a solvent. As the solvent, the one that is used in the above addition reaction may be used as it is.
A reaction product thus obtained may be purified by distillation, crystallization, column-separation and the like, and the purification method may be selected depending on the nature of the product and the kind of impurities.

### [Synthesis method (3) for an adamantane derivative represented by the general formula (I)]

An adamantane derivative represented by the above general formula (I) may be synthesized by hydrogenation of adamantanephenol glycidyl ethers such as 4-(1-adamantyl)-1,3-digylcidyloxybenzene and the like.
The reaction temperature of the above hydrogenation is usually about 20 to about 150°C, and preferably 40 to 100°C. When the reaction temperature is 20°C or above, the reaction rate does not decrease and remains moderate, thus the reaction time is shortened. In addition, when the reaction temperature is 150°C or below, hydrogenation of the intended adamantane derivative is suppressed. Applied hydrogen pressure at the reaction is about 1 to about 30 MPa, and preferably 3 to 10 MPa. When the reaction pressure is 30 MPa or less, special equipment is not necessary as the safety is secured, thus it is useful from an industrial viewpoint. The reaction time is usually about 1 minute to about 24 hours, and preferably 1 to 10 hours.

The above reaction is carried out usually in the presence of a rhodium catalyst or a ruthenium catalyst. As the rhodium catalyst, examples include rhodium supported on activated carbon or alumina, a rhodium oxide and the like. As the ruthenium catalyst, examples include ruthenium supported on activated carbon or alumina, a ruthenium oxide, ruthenium black and the like.
The ratio of a rhodium catalyst or a ruthenium catalyst used, in terms of the amount of rhodium or ruthenium, is about 0.01 to about 10% by mass, and preferably 0.05 to 5% by mass, relative to a raw material monomer. When the ratio of these catalysts used is 0.01% by mass or more, sufficient activity may be obtained, and from the viewpoint of activity enhancement, the ratio of 10% by mass or less is sufficient and it is practical also in terms of economy.
The reaction is carried out in the presence of a solvent. As the solvent, those solvents which are stable under the above reaction conditions are used, and from the viewpoint of solubility of a raw material monomer, an ether solvent and an ester solvent are preferable. Specific examples include tetrahydrofuran, ethyl acetate and the like. They may be used singly or in a combination of two or more kinds.
A reaction product may be purified by distillation, crystallization, column-separation and the like, and the purification method may be selected depending on the nature of the product and the kind of impurities.

### [Synthesis method of an adamantane derivative represented by the general formula (IV) or the general formula (V)]

An adamantane derivative of the present invention as represented by the general formula (IV) or the general formula (V) may be synthesized, without purifying an intended substance from reaction products obtained in the above Synthesis Methods (1) and (2), by further reacting the intended substance or an epoxy group of the intended substance with a raw material non-aromatic adamantane and an alkyl-containing cyclic ether compound or an epihalohydrin compound.

The above reaction is carried out usually at a temperature of about 0 to about 200°C, and preferably at 20 to 150°C. When the reaction temperature is within the above range, the reaction rate does not decrease and remains moderate, thus the reaction time is shortened and the formation of a by-product derived from a halogen-containing substance may be suppressed. The applied absolute pressure at the reaction is about 0.01 to about 10 MPa, and preferably normal pressure to 1 MPa. When the reaction pressure is within the above range, special equipment is not necessary as the safety is secured, thus it is useful from an industrial viewpoint. The reaction time is usually about 1 minute to about 24 hours, and preferably 1 to 10 hours. When the reaction time is within the above range, the amount of an unreacted raw material may be reduced, leading to increase in the reaction yield and manufacturing efficiency.
Further, an alkyl-containing cyclic ether compound is reacted with a non-aromatic adamantane derivative with the amount of the former being 0.9- to 1.5-fold equivalent, and preferably 1.0- to 1.2-fold equivalent, relative to an equivalent of carboxyl group (or a hydroxyl group) of the latter. When the amounts are within the above range, the reaction yield is increased and the formation of a chlorine-containing substance may be suppressed.

The above reaction is carried out usually in the presence of a base catalyst. As the base catalyst, the above-mentioned ones may be used preferably.
The ratio of a base catalyst used is about 1.0 to about 1.5 equivalents, and preferably 1.0 to 1.2 equivalents, relative to a hydroxyl group and a carboxyl group of a raw material monomer. When the ratio of a catalyst used is within the above range, there exists no residual alkyl-containing cyclic ether compound not subjected to a glycidyl formation reaction, thus the effect of catalyst used is sufficiently realized.

The above addition reaction is carried out in the absence or presence of a solvent. As the solvent, the above-mentioned ones may be preferably used. The amount of the solvent to be used is such that the concentration of the above non-aromatic adamantane in it is 0.5% by mass or more, and preferably 5% by mass or more, wherein the adamantane may exist in the state of suspension but preferably in the state of solution.

### [A resin composition]

A resin composition of the present invention is composed of an adamantane derivative of the present invention, but a resin mixture of an adamantane derivative of the present invention and other publicly known epoxy resins may also be used in the resin composition of the present invention. Examples of the publicly known epoxy resins used for the mixture include a bisphenol A epoxy resin, a bisphenol F epoxy resin (bisphenol A diglycidyl ether, bisphenol AD diglycidyl ether, bisphenol S diglycidyl ether, hydrogenated bisphenol A diglycidyl ether, bisphenol F diglycidyl ether, bisphenol G diglycidyl ether, tetramethylbisphenol A diglycidyl ether, bisphenol AF diglycidyl ether, bisphenol C diglycidyl ether and the like), novolak epoxy resins such as a phenol novolak epoxy resin, a cresol novolak epoxy resin and the like, an alicyclic epoxy resin, nitrogen-containing cyclic epoxy resins such as triglycidyl isocyanurate, a hydantoin epoxy resin and the like, a hydrogenated bisphenol A epoxy resin, an aliphatic epoxy resin, a glycidyl ester epoxy resin, a bisphenol S epoxy resin, a biphenyl epoxy resin and a dicyclo-type cyclic epoxy resin, which are a mainstream of a low water-absorption curing type, a naphthalene epoxy resin, polyfunctional epoxy resins such as trimethylolpropane polyglycidyl ether, glycerol polyglycidyl ether, pentaerythritol polyglycidyl ether and the like, fluorine-containing epoxy resins such as a bisphenol AF epoxy resin, and the like, a (meth)acrylate diglycidyl ester, and others. Among them, an epoxy resin not having an aromatic ring is preferable. These may be used singly or in a combination of two or more kinds.

The above epoxy resin may be solid or liquid at room temperature, but generally the one having an average epoxy equivalent of 200 to 2000 is preferable. When the epoxy equivalent is 200 or more, a cured epoxy resin composition is not brittle and has suitable strength. Also, when the epoxy equivalent is 2000 or less, the glass transition temperature (Tg) of the cured product thereof is not low and thus suitable.
In a resin mixture of the above adamantane derivative with the above epoxy resin, the content of the former is preferably 5.0% by mass or more, and far preferably 10% by mass or more. When the content of the former is 5.0% by mass or more, optical characteristics, long-term heat resistance, and electric characteristics of a resin composition of the present invention become sufficient.

### [A curing agent for an epoxy resin: a polymerization initiator]

A resin composition of the present invention may be cured by cationic polymerization using a cationic polymerization initiator as a curing agent for an epoxy resin, or by a reaction using curing agents such as an acid anhydride-type, an amine-type and the like.
As the cationic polymerization initiators, those initiators which may react with an epoxy group or an oxetanyl group by heat or UV light may be used. Examples of such initiators include aromatic diazonium salts such as p-methoxybenzenediazonium hexafluorophosphate and the like; aromatic sulfonium salts such as triphenylsulfonium hexafluorophosphate and the like; aromatic iodonium salts such as diphenyliodonium hexafluorophosphate and the like; an aromatic iodosyl salt, an aromatic sulfoxonium salt, a metallocene compound and the like. Among them, aromatic sulfonium salts such as triphenylsulfonium hexafluorophosphate and the like, aromatic iodonium salts such as diphenyliodonium hexafluorophosphate and the like may be most suitable. They may be used singly or in a combination of two or more kinds.
The amount of the cationic polymerization initiator used is preferably 0.01 to 5.0 parts by mass, and far preferably 0.1 to 3.0 parts by mass, relative to 100 parts by mass of the above adamantane derivative or the above resin mixture (hereinafter sometimes referred to as "Resin Component"). By choosing the content of the cationic polymerization initiator within the above range, suitable polymerization is achieved and good physical properties, namely good optical characteristics and the like may be obtained.

### [A curing agent for an epoxy resin; a curing agent]

In the present invention, as a curing agent, an acid anhydride type curing agent, a phenolic type curing agent, an amine type curing agent and the like may be concurrently used depending on the purpose. By reacting a fluorine-containing adamantane derivative of the present invention having excellent heat resistance and transparency with a curing agent, light resistance, dielectric constant and the like, in addition to heat resistance and transparency, may be improved, and enough solubility for the practical use may be imparted.
Examples of the acid anhydride type curing agents include phthalic anhydride, maleic anhydride, trimellitic anhydride, pyromellitic anhydride, haxahydrophthalic anhydride, tetrahydrophthalic anhydride, methylnadic anhydride, nadic anhydride, glutaric anhydride, methylhexahydrophthalic anhydride, methyltetrahydrophthalic anhydride and the like. Among them, haxahydrophthalic anhydride, tetrahydrophthalic anhydride, methylhexahydrophthalic anhydride, and methyltetrahydrophthalic anhydride are most suitable. They may be used singly or in a combination of two or more kinds.

Furthermore, when an acid anhydride type curing agent is used, a curing accelerating agent may be blended to it for the purpose of accelerating curing. The curing accelerating agent is not particularly restricted so far as it accelerates a reaction between an epoxy resin and its curing agent. Examples of such curing accelerating agents may include phosphorous compounds such as triphenylphosphine, tributylphosphine, tris(p-methylphenyl)phosphine, tris(p-methoxyphenyl)phosphine, tris(p-ethoxyphenyl)phosphine, triphenylphosphine-triphenylborate, tetraphenylphosphonium-tetraphenylborate, tetraphenylphosphonium bromide, tetra-n-butylphosphonium-o,o-diethylphosphorodithioate and the like; tertiary amines such as 1,8-diazabicyclo[5.4.0]undecene-7, triethylenediamine, tris(2,4,6-dimethylaminomethyl)phenol, benzyldimethylamine, triethyleneammonium triphenylborate and the like; imidazoles such as 2-methylimidazole, 2-ethylimidazole, 2-ethyl-4-methylimidazole and their ethylisocyanate compounds, 2-phenylimidazole, 2-phenyl-4-methylimidazole, 2-phenyl-4-methyl-5-hydroxymethylimidazole, 2-phenyl-4,5-dihydroxymethylimidazole and the like; octyl acid salts such as tin octylate, zinc octylate and the like; a quaternary ammonium salt, an organometallic salt, their derivatives and the like.
Among these curing accelerating agents, a phosphorous compound, a tertiary amine, and an imidazole are used preferably.

Examples of the phenolic type curing agents include a phenol/novolak resin, a cresol novolak resin, a bisphenol A novolak resin, a triazine-modified phenol novolak resin and the like. Examples of the curing agents of an amine type include dicyandiamide and aromatic diamines such as m-phenylenediamine, 4,4'-diaminodiphenylmethane, 4,4'-diaminodiphenylsulfone, m-xylylenediamine and the like. They may be used singly or in a combination of two or more kinds.
Among these curing agents, an acid anhydride type curing agent is preferable in view of physical properties such as transparency and the like of a cured resin, and particularly haxahydrophthalic anhydride, tetrahydrophthalic anhydride, methylhexahydrophthalic anhydride, and methyltetrahydrophthalic anhydride are most preferable.
The content of the curing accelerating agent is preferably 0.01 to 8.0 parts, and far preferably 0.1 to 3.0 parts by mass, relative to 100 parts by mass of the above resin component. By choosing the content of the curing accelerating agent in the above range, sufficient curing accelerating effect may be obtained, and coloring of a cured product is not observed.

The blending ratio of a resin component to a curing agent is determined by the ratio of a glycidyl-reactive functional group of the curing agent. The ratio is usually 0.5 to 1.5 equivalents, and preferably 0.8 to 1.2 equivalents of the functional group of the corresponding curing agent, relative to one glycidyl equivalent. By choosing the blending ratio between the resin composition and the curing agent in the above range, there is no slowing of a curing rate of a resin composition, nor lowering of glass transition temperature of a cured resin and lowering of humidity resistance, thus it is suitable.

### [An additive]

Further, in a resin composition of the present invention, many kinds of publicly known additives that have been conventionally used may be blendedas appropriate, if necessary. Examples of such additives include a decay-preventing agent, a modifying agent, a silane coupling agent, a defoaming agent, inorganic powders, a solvent, a leveling agent, a mold-release agent, a dye, pigments and the like.

A resin composition of the present invention is excellent in heat resistance and transparency, and in order to maintain these properties a decay-preventing agent may be added. Examples of the agents include publicly known decay-preventing agents such as a phenolic compound, an amine compound, an organic sulfur compound, a phosphorous compound and the like.
Examples of the phenolic compounds include commercially available materials such as Irganox 1010 (trademark, manufactured by Ciba Specialty Chemicals Inc.), Irganox 1076 (trademark, manufactured by Ciba Specialty Chemicals Inc.), Irganox 1330 (trademark, manufactured by Ciba Specialty Chemicals Inc.), Irganox 3114 (trademark, manufactured by Ciba Specialty Chemicals Inc.), Irganox 3125 (trademark, manufactured by Ciba Specialty Chemicals Inc.), Irganox 3790 (trademark, manufactured by Ciba Specialty Chemicals Inc.), BHT, Cyanox 1790 (trademark, manufactured by Cyanamid Co.), Sumilizer GA-80 (trademark, manufactured by Sumitomo Chemical Co., Ltd.), and the like.

Examples of the amine compounds include such compounds as Irgastab FS042 (trademark, manufactured by Ciba Specialty Chemicals Inc.), GENOX EP (trademark, manufactured by Crompton Corp., chemical name: dialkyl-N-methylamine oxide) and the like; and such hindered amines as ADK STAB LA-52, LA-57, LA-62, LA-63, LA-67, LA-68, LA-77, LA-82, LA-87, and LA-94, all manufactured by Asahi Denka Co., Ltd., Tinuvin 123, 144, 440, 662, Chimassorb 2020, 119, and 944, all manufactured by Ciba Specialty Chemicals Inc., Hostavin N30 manufactured by Hoechst GmbH, Cyasorb UV-3346 and UV-3526, both manufactured by Cytec Industries Inc., Uval 299 manufactured by Great Lakes Chemical Corp., Sanduvor PR-31 manufactured by Clariant Corp., and the like.
Examples of the organic sulfur compounds include such commercially available products as DSTP (Yoshitomi) (trademark, manufactured by Yoshitomi Pharmaceutical Co., Ltd.), DLTP (Yoshitomi) (trademark, manufactured by Yoshitomi Pharmaceutical Co., Ltd.), DLTOIB (trademark, manufactured by Yoshitomi Pharmaceutical Co., Ltd.), DMTP (Yoshitomi) (trademark, manufactured by Yoshitomi Pharmaceutical Co., Ltd.), Seenox 412S (trademark, manufactured by Shipro Kasei, Ltd.), Cyanox 1212 (trademark, manufactured by Cyanamid Co.) and the like.

Examples of the modifying agents include publicly known modifying agents such as a glycol, a silicone, an alcohol and the like. Examples of the silane coupling agents include publicly known silane coupling agents such as a silane-type, a titanate-type and the like. Examples of the defoaming agents include publicly known defoaming agents such as a silicone type and the like. Examples of the inorganic powders include publicly known inorganic powders such as glass powders, silica powders, titania, zinc oxide, alumina and the like having a particle diameter of several nm to 10 µm depending on their use. Examples of the solvents that may be used for epoxy resin powders and as a diluent solvent for coating include aromatic solvents such as toluene, xylene and the like, and ketone solvents such as methyl ethyl ketone, methyl isobutyl ketone, cyclohexanone and the like.

A resin composition of the present invention, obtained by mixing the above resin component, a curing agent or a cationic polymerization initiator, and various additives, is charged into a mold (resin mold) or formed to an intended shape by coating, then cured by heating or irradiating a UV beam. In the case of thermal curing, curing temperature is usually about 50 to about 200°C, and preferably 100 to 180°C. By choosing the temperature at 50°C or above, poor curing does not occur, nor do coloring and the like at 200°C or below. The curing time is dependent on a resin component, a curing agent, a curing accelerating agent, and an initiator to be used, but preferably 0.5 to 6 hours.
The irradiation strength of a UV beam is usually about 500 to about 5000 mJ/cm², and preferably 1000 to 4000 mJ/cm². Heating may be performed after the UV beam irradiation, preferably at 70 to 200°C for 0.5 to 12 hours.
Molding methods may be an injection molding, a blow molding, a press molding and the like, and not particularly restricted, but an injection molding, using a resin composition in pellet form and injection molding equipment, may be preferably used.

A resin obtained by curing a resin composition of the present invention is excellent in heat resistance and transparency, wherein the total light transmittance may reach 70% or more. In addition, as will be shown by later Examples, it is excellent in processability because of low melting point, and gives a cured product having a high glass transition temperature, excellent durability (in heat resistance and light resistance), electric characteristics such as dielectric constant and the like.
As is seen in the above, a resin composition of the present invention has excellent characteristics, thus is suitably used as a resin (a sealant and an adhesive) for an optical semiconductor (an LED and the like), a flat panel display (an organic EL device, a liquid crystal and the like), an electronic circuit and an optical circuit (an optical waveguide), and for optoelectronic components such as a lens for optical communication, an optical film, and others.
Further, a resin composition of the present invention may be used also for a semiconductor element/an integrated circuit (an IC and the like), an individual semiconductor (a diode, a transistor, a thermistor and the like), an LED (an LED lamp, a chip LED, a light receiving element, a lens for an optical semiconductor), a sensor (a temperature sensor, a light sensor, a magnetic sensor), a passive component (a high frequency device, a resistor, a condenser and the like), a structural component (a connector, a switch, a relay and the like), an automobile part (a circuit system, a control system, sensors, a lamp seal and the like), an adhesive (an optical component, an optical disk, a pickup lens) and others, and, in addition, for an optical film and the like as surface coating.
Therefore, the present invention provides a sealant made of the above-mentioned resin composition, for an optical semiconductor, for electronic circuits such as an organic EL device and the like, for optoelectronic components such as an optical waveguide, for a lens for optical communication, for an optical film and the like, and an adhesive used for them.

A composition as a sealant for an optical semiconductor (LED and the like) may be applied to a device of a bombshell type, a surface mount type (SMT) and the like, may adhere well with semiconductors such as GaN formed on a metal or a polyamide, and furthermore may be used by dispersing fluorescent dyes such as YAG and the like in it. Further, it may be used also for a surface coating material of a bomb shell type LED and for a lens of a SMT type LED, and others.
A composition for an organic EL is applicable to the organic EL device having a composition of anode/hole-injection layer/luminescent layer/electron-injection layer/cathode which is formed in this order on the transparent substrates such as generally used glasses, transparent resins and the like. It may be used as an adhesive in covering an organic EL device with a metal can, a metal sheet or a resin film coated with SiN and the like as a sealant of an organic EL device or may directly seal an organic EL device by dispersing inorganic fillers and the like in a resin composition of the present invention in order to impart gas-barrier properties. It may be applied to a bottom emission type, which is currently a mainstream as a display system, but the effects of transparency and heat resistance of a resin composition of the present invention may be advantageously utilized when applied to a top emission type, which will draw attention in the future in view of the light extraction efficiency.
A composition for an electronic circuit is applicable as an interlayer insulation film, as an adhesive between a polyimide for a flexible printed board and a copper foil, or as a resin for a substrate.
A composition for an optical circuit is applicable to a thermooptic switch and an arrayed waveguide grating for a single-mode and a multi-mode, to an optical multiplexer/demultiplexer, to a wavelength-variable filter, or to a core material and a clad material for an optical fiber. It is also applicable to a micro lens array focusing a light to a waveguide and to a mirror of an MEMS-type optical switch. Furthermore, it is applicable also to a dye binder and the like for a photoelectric transducer.
A composition for an optical film is applicable as a display of film substrates for liquid crystal and for organic EL, or as a light diffusion film, an anti-reflection film, a color-converting film using dispersion of a fluorescent dye and the like, and others.

### Examples

Next, the present invention will be explained in further detail, but the invention is not restricted at all by these Examples. In the following Examples and Comparative Examples, the resin compositions obtained are evaluated in the following manner.

### (1) Glass transition temperature

By using a differential scanning calorimeter (DSC-7, manufactured by PerkinElmer, Inc.), 10 mg of a sample was kept at 50°C for 5 minutes under nitrogen atmosphere and then heated at 20°C/minute. A discontinuous point observed in the thermal flux curve thus obtained was taken as a glass transition temperature Tg.

### (2) Total light transmittance

A specimen of 3mm thickness of a sample was measured in accordance with the procedure in JIS K7105 (unit %). A spectrophotometer UV-3100S, manufactured by Shimadzu Corporation, was used as the measuring instrument.

### (3) Light resistance measurement

By using Suntest CPS+, manufactured by Toyo Seiki Seisaku-Sho, Ltd., a sample was irradiated at 60°C by light for 500 hours, and the change of the light transmittance at 400 nm before and after the irradiation was measured by using a sunshine tester.

### (4) Long-term heat resistance test

The change of the light transmittance at 400 nm before and after the test in an oven controlled at 140°C for 100 hours was measured by a sunshine tester. When the transmittance was decreased by 20% or more, it was rated as "poor".

### Production Example 1 (Synthesis of 1-adamantylmalonate diglycidyl ester)

In a flask having an inner volume of 100 ml, equipped with a reflux condenser, a dropping funnel, a stirrer, and a thermometer, were charged 1.5 g (6.3 mmol) of 1-admantylmalonic acid, 30 g (324 mmol) of epichlorohydrin, and 0.15 g of tetraethylammonium bromide, and they were heated until reflux with stirring. Under reflux, 1.25 g (15.6 mmol) of 50% by mass of aqueous sodium hydroxide was added dropwise into the reaction mixture during a period of 30 minutes. After the addition, the reflux was continued for further 3 hours to complete the reaction. The mixture was cooled to room temperature, to which was added 30 ml of water. The resulting mixture was extracted by 100 ml of ethyl acetate. The extract was concentrated to obtain 2.4 g of colorless transparent solution. The solution was purified by a silica gel column to obtain 1.6 g (72% yield, 97% purity by gas chromatography) of 1-adamantylmalonate diglycidyl ester.
This 1-adamantylmalonate diglycidyl ester was identified by nuclear magnetic resonance spectra (¹H-NMR and ¹³C-NMR) and GC-MS. The nuclear magnetic resonance spectra were measured in chloroform-d₆ solvent by JNM-ECA500 manufactured by JEOL Ltd., and the GC-MS was measured by GCMS-QP2010 manufactured by Shimadzu Corporation.

¹H-NMR (500 MHz): 1.62 (m, 6H), 1.82 (s, 6H), 1.94 (s, 3H), 2.61 (m, 2H), 2.78 (m, 2H), 3.15 (m, 2H), 3.15 (s, 1H), 3.92 (m, 2H), 4.42 (m, 2H).
¹³C-NMR (125 MHz): 28.6, 36.2, 36.6, 39.9, 44.6, 49.2, 62.2, 65.1, 65.4, 67.2
GC-MS (EI): 350 (1.2%), 277 (3.9%), 249 (6.7%), 174 (10.1%), 135 (100.0%), 107 (6.1%), 93 (11.3%), 79 (13.0%)

### Example 1

A charge of 1 g (175 epoxy equivalents) of 1-adamantylmalonate diglycidyl ester obtained in Production Example 1, 0.96 g of methylhexahydrophthalic anhydride (manufactured by New Japan Chemical Co., Ltd., trade name MH700) as an acid anhydride type curing agent, and 0.01 g of 1,8-diazabicyclo[5.4.0]undecene-7 (manufactured by San-Apro Ltd., trade name SA102) as a curing accelerator was blended at room temperature, defoamed, and then heated at 120°C for 2 hours and at 150°C for 2 hours to prepare a cured resin (sheet thickness of 3mm). The cured product was evaluated by the above-mentioned methods. The evaluation results are shown in Table 1.

### Comparative Example 1

A cured product was prepared in the same manner as Example 1, except that a bisphenol A epoxy resin (185 epoxy equivalents) instead of 1-adamantylmalonate diglycidyl ester and 0.91 g of methylhexahydrophthalic anhydride were used. The cured product thus obtained was evaluated by the above-mentioned methods. The evaluation results are shown in Table 1.

### Comparative Example 2

A cured product was prepared in the same manner as Example 1, except that a hydrogenated bisphenol A epoxy resin (204 epoxy equivalents) instead of 1-adamantylmalonate diglycidyl ester and 0.82 g of methylhexahydrophthalic anhydride were used. The cured product thus obtained was evaluated by the above-mentioned methods. The evaluation results are shown in Table 1.

### Production Example 2 (Synthesis of 2-(1-adamantyloxymethyl)-2-methylpropane-1,3-diol diglycidyl ether)

In a three-necked flask having an inner volume of 2000 ml, equipped with a reflux condenser, a dropping funnel, a stirrer, and a thermometer, were charged 60.9 g (400 mmol) of 1-admantanol, 60.7 g (600 mmol) of triethylamine, and 600 mL of tetrahydrofuran, and then the mixture was cooled to 0°C by immersing it in an ice bath. The reaction was carried out for 1 hour by adding 55.0 g (480 mmol) of methanesulfonyl chloride in such a way not to exceed 10°C. After bringing the reaction mixture back to room temperature, 960 g (8000 mmol) of trimethylolethane and 60.7 g (600 mmol) of triethylamine were added, and then the mixture was heated under reflux for 3 hours. Then tetrahydrofuran solvent was removed by distillation, a large volume of water was added to the remaining solution, and the resulting mixture was extracted twice by ethyl acetate. After the ethyl acetate solution was thoroughly washed with water, it was concentrated to obtain 80 g of 2-(1-adamantyloxymethyl)-2-methylpropane-1,3-diol as pale yellowish transparent solution (79% yield, 98% purity by gas chromatography).
In the similar manner as the Production Example 1 except that 2-(1-adamantyloxymethyl)-2-methylpropane-1,3-diol was used instead of 1-adamantylmalonic acid, 81 g of the intended 2-(1-adamantyloxymethyl)-2-methylpropane-1,3-diol diglycidyl ether was obtained (55% yield, 96% purity by gas chromatography).

### Example 2

A charge of 1 g (198 epoxy equivalents) of 2-(1-adamantyloxymethyl)-2-methylpropane-1,3-diol diglycidyl ether obtained in Production Example 2, 0.85 g of methylhexahydrophthalic anhydride (manufactured by New Japan Chemical Co., Ltd., trade name MH700) as an acid anhydride type curing agent, and 0.01 g of 1,8-diazabicyclo[5.4.0]undecene-7 (manufactured by San-Apro Ltd., trade name SA102) as a curing accelerator was blended at room temperature, defoamed, and then heated at 120°C for 2 hours and at 150°C for 2 hours to obtain a cured resin (sheet thickness of 3mm). The cured product was evaluated by the above-mentioned methods. The evaluation results are shown in Table 1.

### Production Example 3

In a flask having an inner volume of 100 ml, equipped with a reflux condenser, a dropping funnel, a stirrer, and a thermometer, were charged 1.5 g (6.3 mmol) of 1-admantylmalonic acid, 30 g (324 mmol) of epichlorohydrin, and 0.15 g of tetraethylammonium bromide, and the mixture was heated until reflux with stirring. Under reflux, 1.25 g (15.6 mmol) of 50% by mass of aqueous sodium hydroxide was added dropwise into the reaction solution during a period of 30 minutes. After the dropwise addition, the reflux was continued for further 3 hours to complete the reaction. The mixture was cooled to room temperature, to which was added 30 ml of water. The resulting solution was extracted by 100 ml of ethyl acetate. The extract was concentrated to obtain 2.4 g of colorless transparent solution. The solution was purified by a silica gel column to obtain 0.4 g (yield 18%) of oligomer of 1-adamantylmalonate diglycidyl ester.

### Production Example 4 (Synthesis of 4-(1-adamantyl)-1,3-diglycidyloxycyclohexane)

In an autoclave having an inner volume of 100 ml were charged 8.0 g of 4-(1-adamantyl)-1,3-digylcidyloxybenzene (191 epoxy equivalents), 0.5 g of a rhodium catalyst (manufactured by N.E. Chemcat Corp., trade name 5% Rh Carbon), and 20 g of tetrahydrofuran, and then the atmosphere of the system was replaced with hydrogen gas. The reaction was carried out for about 5 hours with stirring at 50°C under the hydrogen pressure of 4 MPa until lowering of the hydrogen pressure stopped. After the reaction, the catalyst was removed by filtration and then the solvent by distillation to obtain the intended product (yield 72%). The ratio of ring-hydrogenation in the product was found to be 99% as measured by the decrease of a UV absorbance (at the wavelength of 275 nm). The epoxy equivalent of the intended product was 216 and the ratio of the remaining epoxy was 90%.
The product obtained was identified by nuclear magnetic resonance spectra (¹H-NMR and ¹³C-NMR). The nuclear magnetic resonance spectra were measured in DMSO-d₆ solvent by JNM-ECA500 manufactured by JEOL Ltd.

¹H-NMR (500 MHz): 1.29 (m, 9H), 1.48 (m, 10H), 1.95 (m, 3H), 2.69 (dd, 1H), 2.76 (dd, 1H), 2.79 (m, 2H), 2.83 (dd, 1H), 2.87 (dd, 1H), 3.30 (m, 1H), 3.37 (m, 1H), 3.80 (dd, 1H), 3.87 (dd, 1H), 4.26 (dd, 1H), 4.32 (dd, 1H).
¹³C-NMR (125 MHz): 17.4, 28.5, 33.1, 35.7, 36.6, 39.0, 40.5, 43.4, 43.5, 49.7, 49.8, 54.5, 68.8, 68.9, 76.2, 83.5.

### Example 3

A charge of 5 g of 4-(1-adamantyl)-1,3-diglycidyloxycyclohexane obtained in Production Example 4, 3.78 g of methylhexahydrophthalic anhydride (manufactured by New Japan Chemical Co., Ltd., trade name MH700) as an acid anhydride type curing agent, and 0.10 g of 1,8-diazabicyclo[5.4.0]undecene-7 (manufactured by San-Apro Ltd., trade name SA102) as a curing accelerator was blended at room temperature, defoamed, and then heated at 120°C for 2 hours and at 150°C for 2 hours to obtain a cured resin (sheet thickness of 3mm). The cured product was evaluated by the above-mentioned methods. The evaluation results are shown in Table 1.

**[Table 1]**

| | Example 1 | Example 2 | Example 3 | Comparative Example 1 | Comparative Example 2 |
|---|---|---|---|---|---|
| Glass transition temperature (°C) | 135 | 130 | 146 | 130 | 105 |
| Total light transmittance (%) | 92 | 93 | 91 | 80 | 92 |
| Light resistance test (decrease rate %) | good | good | good | poor | good |
| Heat resistance test (decrease rate %) | good | good | good | good | poor |

### Industrial Applicability

An adamantane derivative and a resin composition containing the derivative thereof of the present invention give a cured product which is excellent in optical characteristics such as transparency and light resistance, long-term heat resistance, and electric characteristics such as dielectric constant, thus can be suitably used as a sealant for electronic circuits such as an optical semiconductor, an organic EL device and the like, as optoelectronic components such as an optical waveguide, a lens for optical communication, an optical film and the like, and as an adhesive for them. In addition, they are useful for an organic EL device, a liquid crystal display and the like, illumination devices such as an LED and the like, a coating material for information communication components such as an optical circuit, a sealant, an adhesive and the like.

## Claims

1. An adamantane derivative represented by the following general formula (I): 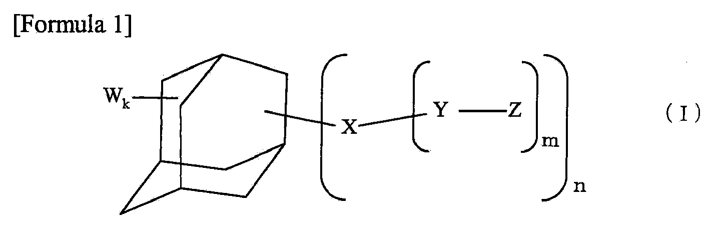 wherein Z represents a group selected from the groups represented by the following general formula (II), 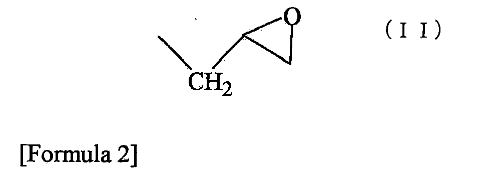 or the following general formula (III) 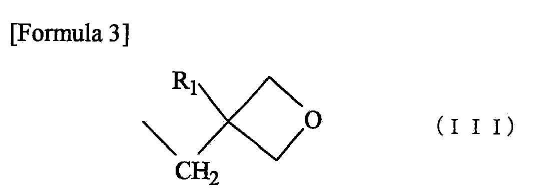 wherein R₁ represents a hydrogen atom or an alkyl group having 1 to 4 carbon atoms, X represents a linking group which may contain an oxygen atom and /or a sulfur atom and is composed of an (m + 1)-valent hydrocarbon, a part or all of the hydrogen atoms in a hydrocarbon as the linking group may be replaced with fluorine atoms, Y represents a group selected from -CO₂ and -O-, W represents a group selected from a hydrogen atom, a halogen atom, a hydrocarbon group, a hydroxyl group, a carboxyl group, and =O formed from two Ws combined together; m represents an integer of 2 to 4; n represents an integer of 1 to 4; and k represents an integer of 0 to 16-n.

2. The adamantane derivative according to claim 1, wherein n is 1.

3. The adamantane derivative according to claim 1, wherein the linkage between an adamantane skeleton and X is made by an ether bond.

4. A method for producing the adamantane derivatives according to claim 1, comprising reacting non-aromatic adamantanes and an alkyl-containing cyclic ether compound in the presence of a base catalyst.

5. A method for producing the adamantane derivatives according to claim 1, comprising reacting non-aromatic adamantanes and an epihalohydrin compound by an addition reaction under an acidic condition, followed by a reaction in the presence of a base catalyst.

6. A method for producing the adamantane derivative according to claim 1, wherein a corresponding aromatic adamantane derivative is ring-hydrogenated in the presence of a rhodium catalyst or a ruthenium catalyst.

7. An oligomer of an adamantane derivative represented by the following general formula (IV), 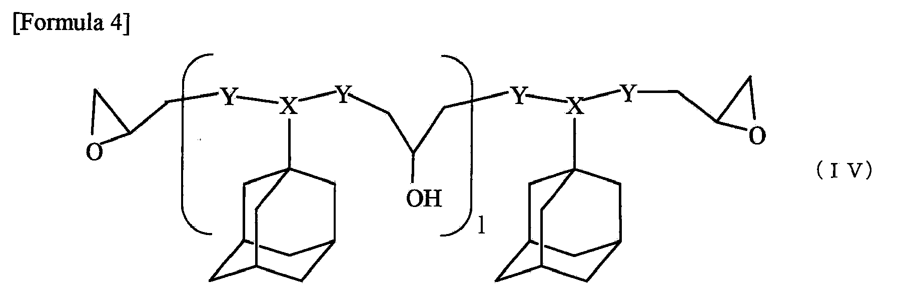 wherein X and Y represent the same as above; I represents an integer of 1 to 4
or by the following general formula (V), 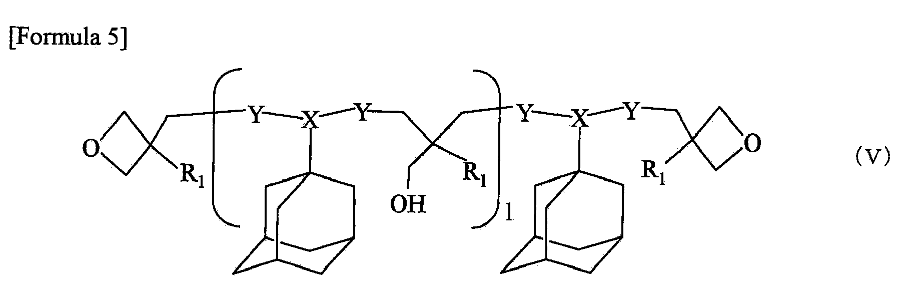 wherein X, Y, and R₁ represent the same as above; and 1 represents an integer of 1 to 4.

8. A resin composition obtained by curing the adamantane derivative according to any of claim 1 or 7 by using a curing agent for an epoxy resin.

9. The resin composition according to claim 6, wherein the curing agent for an epoxy resin is an acid anhydride compound and/or a cationic polymerization initiator.

10. An optoelectronic component using the adamantane derivative according to any of claim 1 or 7.

11. An optoelectronic component using the resin composition according to claim 8.

12. A sealant for an electronic circuit using the adamantane derivative according to any of claim 1 or 7.

13. A sealant for an electronic circuit using the resin composition according to claim 8.
